# FASCICULE DE BREVET EUROPEEN

(11) **EP 3 212 312 B1**
(45) Date de publication et mention de la délivrance du brevet: **22.04.2020**
(21) Numéro de dépôt: 15804873.6
(22) Date de dépôt: 30.10.2015
(51) Int. Cl.: B01D 61/04, B01D 61/16, B01D 61/58, C13B 20/16, C13B 20/18, C13K 13/00, C07H 3/02, C13K 1/04, B01D 61/02, B01D 61/14, B01D 61/42

(54) **PROCEDE DE PURIFICATION D'OSES SANS AJUSTEMENT DE PH**
VERFAHREN ZUR REINIGUNG VON OSEN OHNE ANPASSUNG DES PH-WERTS
METHOD FOR PURIFYING OSES WITHOUT ADJUSTING PH

(30) Priorité: 31.10.2014 FR 1460484
(43) Date de publication de la demande: 06.09.2017
(73) Titulaire: CentraleSupélec, 91192 - Gif sur Yvette Cedex (FR)
(72) Inventeur: THEOLEYRE, Marc-André, 75020 Paris (FR); LEMAIRE, Julien, 08220 Seraincourt (FR)
(74) Mandataire: Cabinet Bleger-Rhein-Poupon
(86) Numéro de dépôt international: PCT/FR2015/052940
(87) Numéro de publication internationale: WO 2016/066978

(56) Documents cités:
- WO-A1-2007/048879
- WO-A1-2010/046532
- JP-A- S5 282 737
- US-A1- 2002 079 268
- US-A1- 2005 056 600
- US-A1- 2012 211 366
- SJOMAN E ET AL: "Nanofiltration of monosaccharide containing solution to recover xylose", DESALINATION, ELSEVIER, AMSTERDAM, NL, vol. 199, no. 1-3, 20 novembre 2006 (2006-11-20), pages 348-349, XP028021074, ISSN: 0011-9164, DOI: 10.1016/J.DESAL.2006.03.082 [extrait le 2006-11-20]

## Description

La présente invention concerne le domaine de la valorisation de la fraction hémicellulosique, qui est issue de la biomasse lignocellulosique.

L'invention concerne plus particulièrement une méthode de purification des pentoses, qui sont des sucres constituant l'hémicellulose, tout en permettant un recyclage du catalyseur acide, ce dernier étant utilisé pour hydrolyser la biomasse lignocellulosique et en extraire la cellulose.

Traditionnellement, la cellulose est utilisée dans la fabrication de matériaux, par exemple le papier, par les industries papetières.

La cellulose peut également être convertie en bioéthanol, par la filière bioéthanol dite « de 2^{nde} génération ».

Cette filière permet d'obtenir des biocarburants à partir de constituants végétaux avantageusement non alimentaires. En d'autres termes, il n'y a pas de concurrence entre cette filière et un usage alimentaire des végétaux, comme cela peut être le cas avec le bioéthanol produit à partir de colza ou de betterave par exemple.

En particulier, les constituants végétaux de départ pouvant être utilisés pour l'obtention de biocarburants de 2^{nde} génération sont, par exemple, le bois, les résidus verts, les pailles de céréales, le fourrage, les résidus forestiers, le miscanthus, la bagasse de canne à sucre, etc., cette dernière correspondant au résidu fibreux de canne à sucre une fois que le suc en a été extrait. Tous ces constituants végétaux peuvent représenter la biomasse lignocellulosique.

La cellulose est le composant majoritaire de cette biomasse lignocellulosique, qui peut comporter jusqu'à 50% de cellulose.

L'extraction de la cellulose à partir de la biomasse lignocellulosique est généralement effectuée par mise en œuvre de techniques physico-chimiques. En particulier, on peut réaliser une étape d'hydrolyse acide de la biomasse lignocellulosique, ce qui permet de récupérer une pâte de cellulose. On effectue ensuite une hydrolyse enzymatique de la cellulose, afin d'obtenir des molécules de glucose, et celui-ci est ensuite transformé en éthanol par fermentation, au moyen de levures.

Outre la cellulose, la biomasse lignocellulosique comporte également de l'hémicellulose et de la lignine.

La lignine consiste en une macromolécule de structure complexe et de haut poids moléculaire.

La structure chimique de la lignine est variable. Les lignines sont plus particulièrement des polymères de monolignols, et il en existe au moins trois différents : l'alcool coumarylique, l'alcool coniférylique et l'alcool sinapylique. La fraction de chacun des monomère dans la lignine varie de façon importante et dépend de différents facteurs, comme la lignée végétale, l'espèce, l'organe, ou encore le tissu.

Pour ce qui est de l'hémicellulose, qui compose la biomasse lignocellulosique pour environ 30% en masse, celle-ci est le 2ème composant principal de la paroi pectocellulosique végétale, après la cellulose. L'hémicellulose joue un rôle de pontage dans cette paroi, entre les fibres de cellulose et d'autres constituants de ladite paroi.

L'hémicellulose consiste en un polysaccharide branché comportant différents types d'osés, à la différence de la cellulose qui est exclusivement constituée par des molécules de glucose, et plus particulièrement par des unités de D-anhydroglucopyranose reliées entre elles par des liaisons glycosidiques β(1→4).

En ce qui concerne les monomères d'oses qui forment l'hémicellulose, ceux-ci peuvent être du glucose, du xylose, du mannose, du galactose, du rhamnose, ou encore de l'arabinose. Le monomère le plus représenté au sein de l'hémicellulose est le xylose.

L'hémicellulose est une molécule fibreuse, insoluble dans l'eau. Majoritairement composée de pentoses, elle est très peu fermentescible et il est donc difficile de synthétiser des biocarburants à partir d'hémicellulose.

Par conséquent, seule la cellulose extraite de la biomasse lignocellulosique est aisément transformable en éthanol.

Il convient toutefois de proposer des techniques permettant de valoriser la fraction hémicellulosique de la biomasse lignocellulosique, c'est-à-dire la fraction comportant l'hémicellulose, et plus particulièrement les oses, notamment les pentoses, qui composent pour partie l'hémicellulose.

En effet, les pentoses, et plus spécifiquement le xylose, présentent un intérêt au niveau de plusieurs applications industrielles, comme la production d'intermédiaires chimiques, de xylitol, de tensio-actifs ou de résines.

Les procédés de purification des pentoses qui sont actuellement mis en œuvre au niveau industriel ont peu évolué depuis la fin des années 1980. Ces procédés présentent notamment l'inconvénient d'être très consommateurs d'eau, de réactifs chimiques, et sont en outre très polluants.

De manière plus précise, une fois l'hydrolyse acide de la biomasse lignocellulosique effectuée pour récupérer la cellulose, le procédé de référence utilisé jusqu'à présent pour permettre une purification des sucres contenus dans l'hydrolysat acide consiste tout d'abord à neutraliser ce dernier.

La neutralisation de l'hydrolysat acide est traditionnellement effectuée par un ajout, à celui-ci, d'une base minérale comme de la chaux ou de la soude. Cette neutralisation va entrainer la précipitation des macromolécules organiques, comme les protéines ou encore la lignine.

Les macromolécules organiques précipitées, ainsi que les matières en suspension, sont ensuite éliminées par décantation ou centrifugation. L'hydrolysat est ensuite déminéralisé par chromatographie, comme décrit dans les documents de brevet US 5 084 104, US 6 239 271 et WO 2010/046532, par échange d'ions, comme décrit dans les documents US 4 075 406, FR 2 655 661 et WO 2004/108739.

Enfin, comme décrit dans les documents US 5 084 104 et WO 2010/046532, l'hydrolysat obtenu peut être concentré dans l'optique d'obtenir des cristaux de xylose d'une grande pureté.

Il est également envisageable que l'hydrolysat ainsi traité soit utilisé pour produire du xylitol, des tensio-actifs, ou encore des résines.

Toutefois, l'inconvénient majeur de ces procédés réside dans le fait qu'ils sont extrêmement polluants, car ils conduisent à rejeter environ 90% des acides qui sont utilisés pour l'hydrolyse de la biomasse.

Des techniques développées plus récemment, et décrites dans les documents de brevet WO 2008/096971 et US 2012/0211366 ont permis de montrer l'intérêt de l'électrodialyse pour permettre la déminéralisation de l'hydrolysat, après neutralisation totale ou partielle de ce dernier.

Plus précisément, le document de brevet US 2012/0211366 est relatif à une méthode de production de xylose à partir d'un hydrolysat, utilisant l'électrodialyse.

Dans cette méthode, après une étape d'hydrolyse de la biomasse végétale, le pH de l'hydrolysat obtenu, initialement de l'ordre de 0,8 à 1,2, doit être ajusté, par ajout d'hydroxyde de sodium, pour arriver à un pH compris entre 1,5 et 2,5. Il en résulte que le catalyseur acide perd la majorité de son pouvoir catalytique et n'est pas susceptible d'être récupéré et recyclé en totalité.

En outre, l'ajout d'hydroxyde de sodium entraine une augmentation de la quantité de sels à séparer lors de l'étape d'électrodialyse. Toutefois, cette étape d'ajustement du pH est essentielle dans le procédé du document US 2012/0211366 car elle permet une précipitation des impuretés solubles à pH acide, telles que les protéines, les pigments et les substances humiques, qui contamineraient les membranes d'électrodialyse. Une étape de clarification est alors nécessaire, pour éliminer les matières en suspension insolubles, par utilisation d'un microfiltre.

Quoiqu'il en soit, dans les procédés connus de l'état de la technique, le catalyseur acide utilisé dans l'hydrolyse de la biomasse est peu ou pas recyclé. En effet, sa neutralisation, ou l'ajustement du pH à une certaine valeur, nécessite un certain montant de base. Par conséquent, la quantité de sels à séparer ensuite lors de l'étape de déminéralisation est considérablement augmentée.

Il en résulte que les procédés existants nécessitent, pour leur mise en œuvre, une quantité très importante d'eau, d'énergie et de réactifs chimiques. Par conséquent, de grandes quantités d'eaux usagées doivent être traitées par la suite.

Le document de brevet WO 2008/096971 a permis de mettre en évidence l'intérêt de l'utilisation de la technique d'électrodialyse dans un procédé destiné à la valorisation des pentoses.

Plus particulièrement, dans ce document, il est question d'un procédé de production de xylitol à partir d'un hydrolysat comprenant notamment du xylose et de l'arabinose, la biomasse de départ consistant en des fruits tropicaux.

Dans le procédé en question, après une étape traditionnelle d'hydrolyse acide de la biomasse, on procède à une précipitation des ions, notamment des ions sulfate et les ions calcium, en augmentant le pH de l'hydrolysat qui passe alors d'un pH 1 ou 2 à un pH pouvant aller jusqu'à 7.

Toutefois, même après cette étape de précipitation, l'hydrolysat est susceptible de contenir encore des ions non précipités. Ces derniers, bien qu'ils soient présents dans l'hydrolysat à faible concentration, peuvent entrainer la formation de dépôt au cours de l'étape ultérieure de concentration des produits d'intérêt, aboutissant finalement à une réduction du rendement dans la production de xylitol.

La mise en œuvre d'une technique d'électrodialyse permet d'éliminer les sels non précipités tout en limitant l'utilisation de résines échangeuses d'ions, nécessitant inévitablement des étapes de régénération desdites résines, coûteuses en temps et en réactifs chimiques.

Cependant, dans le procédé décrit ici, tout comme dans les autres méthodes proposées dans l'état de la technique, une étape d'élévation du pH est nécessaire pour éliminer les macromolécules et certains ions dissouts. Aussi, une grande partie de l'acide ajouté au moment de l'hydrolyse de la biomasse est neutralisé, ce qui entraîne la perte de l'essentiel de son pouvoir catalytique.

En outre, les membranes anioniques ou cationiques utilisées dans la technique d'électrodialyse sont très sensibles au colmatage et à la contamination par des molécules organiques. Par conséquent, la durée de vie et l'efficacité desdites membranes sont susceptibles d'être réduites.

Les macromolécules peuvent également précipiter dans le module d'électrodialyse, ou stack, lorsque le pH est augmenté.

Aussi, en raison du prix élevé des membranes d'électrodialyse, il est nécessaire d'éliminer de manière efficace les composants de l'hydrolysat susceptibles de précipiter, et ce afin de pouvoir mettre en œuvre la technique d'électrodialyse à une échelle industrielle.

Il a déjà été évoqué le fait que les macromolécules, en particulier la lignine et les protéines, sont précipitées par élévation du pH de l'hydrolysat. Celui-ci, étant initialement de l'ordre de 1 au moment de l'hydrolyse acide, doit être ajusté à une valeur comprise entre 2 et 7, par exemple en ajoutant de la chaux ou une solution de soude à l'hydrolysat, ce qui aboutit inévitablement à la perte de l'essentiel du pouvoir catalytique de l'acide.

L'invention offre la possibilité de pallier les divers inconvénients de l'état de la technique en proposant un procédé innovant de valorisation et de purification des sucres, en particulier des pentoses, qui sont présents dans un hydrolysat acide d'hémicellulose, obtenu à partir de biomasse lignocellulosique.

Le procédé selon la présente invention présente l'avantage d'être plus économique et plus « durable » que les méthodes proposées jusqu'à présent, dans le sens où les réactifs chimiques, et notamment le catalyseur acide utilisé pour l'hydrolyse de la biomasse, sont recyclés en grande partie.

A cet effet, la présente invention concerne un procédé de purification d'oses issus de l'hémicellulose provenant de biomasses lignocellulosiques, lesdits oses étant contenus dans un hydrolysat acide obtenu par hydrolyse partielle de biomasses lignocellulosiques au moyen d'au moins un catalyseur acide, ledit hydrolysat acide comportant en outre une matrice cellulosique, des résidus solides et/ou des matières en suspension, ledit procédé étant caractérisé en ce qu'il comporte les étapes suivantes :
- on élimine, de l'hydrolysat acide, la matrice cellulosique et les résidus solides et/ou les matières en suspension pour obtenir un hydrolysat clarifié ;
- sans aucun ajout de réactif chimique basique pour augmenter le pH, on soumet ledit hydrolysat clarifié à au moins une étape d'ultrafiltration et/ou à au moins une étape de nanofiltration, de sorte à obtenir un filtrat contenant la majorité des pentoses et un rétentât contenant les espèces susceptibles de précipiter sous l'effet d'une augmentation du pH ;
- on traite ledit filtrat par au moins une étape d'électrodialyse de sorte à récupérer le catalyseur acide, dans une solution enrichie en acide, et à obtenir un filtrat désacidifié.

Selon une particularité de l'invention, le présent procédé se caractérise également en ce que, préalablement à la filtration, on élimine, dans un premier temps, la matrice cellulosique puis, dans un second temps, les résidus solides et/ou les matières en suspension.

De manière avantageuse, on élimine les résidus solides et/ou les matières en suspension par décantation et/ou centrifugation et/ou filtration sur presse et/ou filtration sur membrane.

Selon un mode de réalisation particulièrement intéressant, le catalyseur acide utilisé consiste en de l'acide sulfurique dont la concentration, dans l'hydrolysat acide, est avantageusement comprise entre 5 et 50 g/L.

Le seuil de coupure du filtre utilisé lors de l'étape de filtration est compris entre 100 et 50000 Da, de préférence entre 10 000 et 15 000 Da.

Par ailleurs, une caractéristique additionnelle du présent procédé est définie par le fait que, lors de l'étape d'électrodialyse, on introduit, dans les compartiments de l'appareil d'électrodialyse où se concentre l'acide de l'eau acidifiée de sorte à avoir une conductivité supérieure ou égale à 5 mS/cm.

La présente invention prévoit également qu'après l'étape d'électrodialyse, on peut également déminéraliser ledit filtrat désacidifié par électrodialyse et/ou par chromatographie et/ou par échange d'ions.

De manière avantageuse, lesdits oses sont des pentoses, de préférence du xylose et/ou de l'arabinose.

Le présent procédé de purification est encore caractérisé en ce que, suite à l'étape de déminéralisation du filtrat désacidifié, on purifie le xylose par concentration du filtrat désacidifié et déminéralisé puis par cristallisation.

Conformément à l'invention, il peut encore être prévu que suite à l'étape de cristallisation, suite à laquelle on obtient une liqueur sucrée comprenant du xylose et au moins un autre ose, on procède à une étape de séparation pour séparer le xylose du (des) autre(s) ose(s).

Dans ce cas, on peut effectuer la séparation du xylose et du (des) autres(s) ose(s) par cristallisation, par chromatographie d'échange de ligand laquelle peut être combinée ou non avec une cristallisation.

La présente invention comporte de nombreux avantages.

D'une part, le procédé permet, sans aucune neutralisation du catalyseur acide au moyen d'un réactif basique, d'éliminer la quasi-totalité des molécules organiques, comme les lignines ou les protéines. Ces molécules ne sont alors plus susceptibles d'intervenir dans la suite du procédé, notamment en colmatant ou en contaminant les membranes ioniques, ou de précipiter lorsque le pH augmente inévitablement au cours de l'étape d'électrodialyse. Ainsi, contrairement aux procédés existants, les membranes d'électrodialyse ont une durée de vie améliorée, ce qui permet d'envisager une transposition du procédé selon l'invention à un niveau industriel.

D'autre part, le présent procédé de purification présente l'avantage de permettre une récupération, par électrodialyse, du catalyseur acide qui est utilisé pour l'hydrolyse de la biomasse lignocellulosique. Il en résulte que le catalyseur acide recyclé peut avantageusement être réutilisé lors d'un procédé ultérieur de purification d'osés. En outre, la concentration en acide peut être aisément réajustée avant sa réutilisation en tant que catalyseur d'hydrolyse de biomasse.

D'un autre côté, les rendements de purification des oses, et notamment des pentoses, obtenus avec le présent procédé, sont très similaires aux rendements des procédés mis en œuvre traditionnellement dans l'état de la technique.

Par conséquent, à performances de purification égales avec les procédés conventionnels, la présente méthode permet une réduction de la consommation d'eau. On réduit également l'utilisation de réactifs chimiques, notamment de catalyseur acide mais également de composé basique, comme la chaux ou la soude, essentiel dans les procédés conventionnels pour l'étape de neutralisation de l'acide. Il en résulte une diminution des coûts de fonctionnement. En outre, l'impact environnemental de l'étape de purification des oses, notamment des pentoses, est considérablement réduit.

Le procédé selon l'invention est donc tout particulièrement intéressant dans le cadre du développement de projets de valorisation de la biomasse lignocellulosique qui soient à la fois rentables et respectueux des principes de développement durable.

D'autres caractéristiques et avantages de l'invention ressortiront de la description détaillée qui va suivre des modes de réalisation non limitatifs de l'invention, en référence aux figures annexées dans lesquelles :

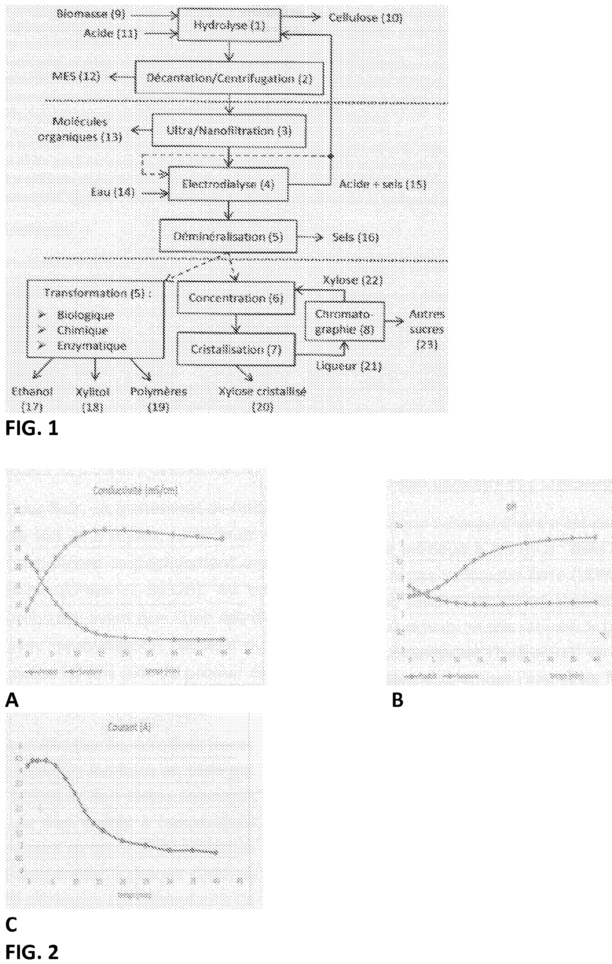

- la figure 1 est un diagramme représentant, de manière schématique, différentes étapes mises en œuvre dans un mode de réalisation préférentiel du procédé de purification des oses selon l'invention ;
- les figures 2A, 2B et 2C sont des graphiques illustrant respectivement l'évolution du pH, de la conductivité (en mS/cm) et de l'intensité (en A) en fonction du temps (en min) lors d'une première phase d'électrodialyse avec 2 L de filtrat, correspondant à l'hydrolysat ayant subi une étape d'ultrafiltration avec une membrane de porosité égale à 10 000 Da et 2 L de saumure « fraîche » correspondant à une solution d'acide sulfurique H₂SO₄ à une concentration massique égale à 0,5 g/L ;

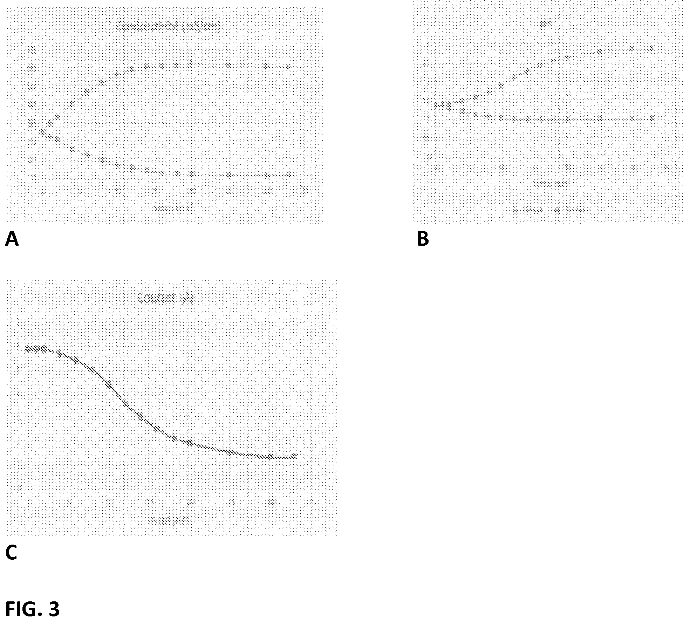

- les figures 3A, 3B et 3C sont des graphiques illustrant respectivement l'évolution du pH, de la conductivité (en mS/cm) et de l'intensité (en A) en fonction du temps (en min) lors d'une deuxième phase d'électrodialyse avec 2,2 L de filtrat, correspondant à l'hydrolysat ayant subi une étape d'ultrafiltration avec une membrane de porosité égale à 10 000 Da et 1,2 L de saumure récupérée après la première phase d'électrodialyse.

Tel que représentée sur la figure 1, la présente invention concerne un procédé de purification d'osés. Au départ, ces oses sont contenus dans des biomasses lignocellulosiqes 9, au sein de macromolécules complexes qui composent ces dernières.

Les biomasses lignocellulosiques de départ 9 dans le présent procédé peuvent comporter tout type de constituants végétaux. Ainsi, les biomasses de départ 9 peuvent être avantageusement composées d'au moins un constituant choisi parmi le bois, les résidus verts, les pailles de céréales, le fourrage, les résidus forestiers, le miscanthus, la bagasse de canne à sucre, etc.

Plus particulièrement, les oses qui sont purifiés par la mise en œuvre du procédé selon l'invention sont des oses qui entrent dans la composition de l'hémicellulose, cette dernière étant l'un des trois principaux constituants des biomasses lignocellulosiques, avec la cellulose et la lignine.

La fraction hémicellulosique dont proviennent ces oses est issue de l'hydrolyse partielle 1 de biomasses lignocellulosiques 9, ladite hydrolyse 1 étant effectuée par la mise en contact entre lesdites biomasses 9 et au moins un catalyseur acide 11.

Ledit catalyseur acide 11 utilisé pour effectuer l'étape d'hydrolyse 1 consiste, selon un mode de réalisation particulier mais non limitatif, en de l'acide sulfurique de formule chimique H₂SO₄.

De préférence, l'acide sulfurique H₂SO₄ présente une concentration massique comprise entre 5 et 50 g/L, préférentiellement entre 10 et 20 g/L, et plus préférentiellement encore une concentration égale à 15 g/L, dans l'hydrolysat acide, ou milieu réactionnel.

Cependant, tout autre type de catalyseur acide apte à permettre l'hydrolyse de biomasses lignocellulosiques peut être utilisé.

L'étape de catalyse acide 1 a pour but premier de permettre la récupération d'une pâte de cellulose 10 à partir d'un hydrolysat acide comportant notamment, en outre, des résidus solides et/ou des matières en suspension. Pour ce qui est de la pâte de cellulose 10, également dénommée matrice cellulosique, celle-ci est utilisée par la suite notamment en industrie papetière ou pour la fabrication de biocarburant de 2^{nde} génération, par exemple du bioéthanol.

Suite à l'hydrolyse acide 1, et après élimination de la cellulose 10, des résidus solides et/ou des matières en suspension, on obtient un hydrolysat acide clarifié particulièrement riche en oses, et notamment en pentoses, provenant de l'hydrolyse de la macromolécule d'hémicellulose. Les pentoses sont des oses, ou monosaccharides, qui comportent 5 atomes de carbone et ont pour formule brute C₅H₁₀O₅. L'hémicellulose est notamment composée d'arabinose et surtout de xylose, qui sont tous deux des aldopentoses, c'est-à-dire qu'ils comportent une fonction aldéhyde en position 1.

Toutefois, il a déjà été évoqué le fait que l'hémicellulose est également constituée par d'autres oses, comme le glucose, le mannose, le galactose et le rhamnose. Ces oses sont tous des hexoses, de formule brute C₆H₁₂O₆, et peuvent également être dissouts dans l'hydrolysat acide.

Il est envisageable d'éliminer simultanément dudit hydrolysat acide, au moyen d'une seule étape de filtration, la matrice cellulosique 10, les résidus solides et/ou les matières en suspension, de sorte à obtenir un hydrolysat clarifié.

Dans un autre mode de réalisation, on élimine dans un premier temps la matrice cellulosique, en la prélevant simplement de l'hydrolysat acide, puis, dans un second temps, on procède à l'élimination des résidus solides et/ou les matières en suspension présent dans l'hydrolysat acide.

Cette élimination des résidus solides et/ou des matières en suspension est effectuée, de manière avantageuse, par la mise en œuvre d'un traitement par décantation et/ou par centrifugation et/ou par filtration sur presse et/ou par filtration sur membrane, cette dernière pouvant consister en une micro-filtration ou en une ultra-filtration.

Une telle étape permet d'éliminer les résidus solides et/ou les matières en suspension 12, ou MES, provenant par exemple des constituants végétaux de départ et qui pourraient éventuellement encore être présents dans l'hydrolysat acide. On obtient alors un hydrolysat clarifié de tous résidus solides et de toutes MES.

Dans la suite du procédé, et comme illustré sur la figure 1 jointe, on effectue au moins une étape d'ultrafiltration et/ou au moins une étape de nanofiltration 3 sur l'hydrolysat acide clarifié. Cette étape a pour fonction d'éliminer, en conditions acides, sans aucune modification du pH, les macromolécules susceptibles de précipiter lorsque le pH sera inévitablement augmenté lors d'une étape ultérieure du procédé.

Cette étape de filtration permet d'obtenir, d'une part, un filtrat, qui correspond au liquide récupéré en sortie de filtration et, d'autre part, un rétentat correspondant à la fraction retenue au niveau du filtre.

La réalisation d'un tel traitement de filtration 3 dudit hydrolysat acide clarifié présente l'avantage substantiel de favoriser l'élimination des molécules organiques, comme la lignine ou d'autres protéines, qui se retrouvent dans le rétentat. En outre, cette élimination est effectuée sans aucun ajout de réactif chimique basique pour augmenter le pH et entrainer une précipitation desdites molécules. Ainsi, le catalyseur acide n'est pas neutralisé et peut être récupéré ultérieurement pour être réutilisé car son pouvoir catalytique est entièrement conservé. En effet, ledit catalyseur est retrouvé au niveau du filtrat, ce dernier contenant également, entre autres, les oses issus d'hémicelluloses qu'il convient de purifier.

L'étape d'ultrafiltration et/ou de nanofiltration est effectuée au moyen d'une membrane, ou filtre, présentant un seuil de coupure compris entre 100 et 50000 Da, et plus préférentiellement encore entre 10000 et 15000 Da.

Les membranes d'ultrafiltration et/ou les membranes de nanofiltration utilisées dans le présent procédé sont des membranes organiques qui ont été soigneusement sélectionnées pour leur résistance à un pH acide de l'ordre de 1 et pour permettre une élimination sélectives des impuretés solubles au pH de l'hydrolyse.

Les seuils de coupure susmentionnés permettent en effet, avantageusement, une élimination optimale de l'essentiel des molécules organiques, susceptibles de précipiter lorsque le pH est augmenté ultérieurement.

En effet, étant donné que ces dernières sont susceptibles de contaminer des membranes ioniques utilisées ultérieurement dans le procédé, voire de précipiter lors de l'étape suivante d'électrodialyse, il est particulièrement important d'éliminer la plus grande proportion possible de ces molécules.

Suite à l'étape de filtration, on procède donc à une étape de traitement du filtrat par une technique d'électrodialyse 4 qui permet, de manière avantageuse, de récupérer la majeure partie du catalyseur acide 11 qui a été utilisé pour hydrolyser la biomasse lignocellulosique 9.

Plus précisément, ledit catalyseur acide 11 est récupéré, au cours de l'étape d'électrodialyse 4 dans une solution enrichie en acide 15, et comportant également des sels, également dénommée « saumure ».

Suite à cette étape d'électrodialyse 4, on récupère également le filtrat qui a été débarrassé de l'acide, et qui est, de ce fait, dénommé filtrat désacidifié dans la suite de la description.

La technique d'électrodialyse permet une extraction d'ions présents dans une solution. Elle est effectuée au moyen d'un électrodialyseur, ou cellule d'électrodialyse, composé d'une pluralité de compartiments séparés par des membranes anioniques et cationiques. Généralement, l'électrodialyseur comporte une alternance de membranes anioniques et cationiques, formant de multiples cellules d'électrodialyse et qui sont positionnées entre deux électrodes permettant la migration des ions sous l'effet d'une différence de potentiel électrique.

Les membranes anioniques comportent des résines à groupes cationiques chargés positivement autorisant le passage des anions de la solution qui peuvent pénétrer dans la membrane et remplacer les anions présents initialement au niveau de la membrane. A l'inverse, les membranes cationiques sont constituées par des groupes anioniques chargés négativement, autorisant de ce fait la pénétration des cations et repoussant les anions sous l'action du champ électrique.

Les cations, qui migrent dans le sens du courant électrique vers la cathode chargée négativement, sortent du premier compartiment en traversant la membrane cationique et sont bloqués dans un deuxième compartiment par la membrane anionique.

Les anions chargés négativement sortent également du premier compartiment en migrant à travers la membrane anionique vers l'anode chargée positivement. Ils sont ensuite bloqués dans un deuxième compartiment par la membrane cationique.

Par conséquent, le premier compartiment voit sa concentration en sel diminuer. C'est la raison pour laquelle il est appelé compartiment de dilution. Au contraire, dans le deuxième compartiment la concentration en ions dissouts augmente ; c'est un compartiment dit de concentration (compartiment de la « saumure »).

L'appareil d'électrodialyse comporte donc une alternance de compartiments de dilution et de compartiments de concentration.

Le catalyseur acide 11 peut alors être récupéré dans les compartiments de concentration de l'électrodialyseur. Plus précisément, et comme déjà évoqué précédemment, on récupère une solution 15 enrichie en acide et comportant, outre le catalyseur acide, également des sels, ladite solution 15 étant également appelée« saumure ».

Afin de pouvoir recycler le catalyseur acide au cours de la première étape d'hydrolyse 1 de la biomasse lignocellulosique 9, la solution 15, enrichie en acide, doit atteindre une concentration suffisante en acide. Dans cette optique, ladite solution 15 peut à nouveau subir au moins une étape d'électrodialyse 4, symbolisée par la flèche en pointillée sur le schéma de la figure 1.

En d'autres termes, la saumure 15 n'est pas renouvelée entre deux étapes d'électrodialyse, tandis qu'un nouveau filtrat, comportant une certaine concentration de catalyseur acide et des pentoses à purifier, est traité par électrodialyse, de façon à simuler un contre-courant.

Ainsi, par le biais de l'électrodialyse, il est envisageable, d'une part, de recycler le catalyseur acide mais également, d'autre part, de réajuster sa concentration pour préparer son recyclage en vue d'une nouvelle étape d'hydrolyse de biomasse lignocellulosique.

Dans l'optique d'ajuster la concentration en acide, il est également envisageable de contrôler les volumes de liquide mis en œuvre au cours de l'électrodialyse, à savoir, d'une part, le volume de filtrat à traiter, et, d'autre part, le volume de solution d'électrodialyse, ou eau 14.

Dans un exemple préférentiel, l'eau 14 qui est introduite initialement dans les compartiments où se concentre l'acide, c'est-à-dire les compartiments de concentration, doit être légèrement acidifiée afin d'avoir une conductivité suffisante, supérieure ou égale à 5 mS/cm. En effet, le procédé d'électrodialyse est basé sur le transport des ions à travers des membranes au moyen d'un champ électrique ; cela suppose que les solutions en circulation soient conductrices.

Préférentiellement, ladite eau 14 présente un pH compris entre 1,5 et 2,5, et plus préférentiellement encore de l'ordre de 2. Cette eau 14 ou « saumure » peut notamment consister en une solution d'acide sulfurique présentant une concentration massique comprise entre 0,25 et 1 g/L, de préférence de l'ordre de 0,5 g/L.

De manière avantageuse, au cours de l'électrodialyse 4, on procède à un suivi du pH dans les différents compartiments de dilution et de concentration de l'appareil d'électrodialyse. Ainsi, ce suivi permet une optimisation du traitement par électrodialyse. Ce suivi permet en outre d'identifier le point où on a récupéré l'essentiel du catalyseur acide 11 de départ, mais avec le moins de sels possible.

Suite à l'étape d'électrodialyse, on procède à une déminéralisation 5 du filtrat désacidifié, de sorte à éliminer une proportion substantielle de sels 16 qui pourraient encore être contenus dans ledit filtrat.

L'étape de déminéralisation 5 peut être de différente nature, selon la voie de valorisation ultérieure qui est choisie pour récupérer les oses issus de la fraction hémicellulosique, notamment les pentoses.

Plus particulièrement, le filtrat désacidifié est déminéralisé par au moins une technique choisie parmi l'électrodialyse, la chromatographie et l'échange d'ions.

En d'autres termes, il est envisageable de mettre en œuvre l'une ou l'autre de ces techniques, ou encore d'utiliser une combinaison de plusieurs de ces techniques, de sorte à obtenir un filtrat à la fois désacidifié et déminéralisé riche en oses, et notamment en pentoses, issus de l'hémicellulose.

La ou les technique(s) de déminéralisation mise(s) en œuvre dépend(ent) de la voie de valorisation des oses qui va être choisie ultérieurement, comme évoqué précédemment, mais également de la nature des sels présents (minéraux ou organiques, monovalents ou divalents) et de leur concentration. Cela va permettre de minimiser la consommation d'eau, d'énergie, et également de réactifs chimiques.

Dans un exemple préférentiel de réalisation, une étape de déminéralisation par électrodialyse ou par chromatographie est mise en œuvre avantageusement pour enlever une proportion importante de sels 16 du filtrat désacidifié. L'échange d'ions sera plus adapté en tant que traitement final dans le but d'éliminer les traces de sels qui pourraient rester.

Suite à l'étape de déminéralisation, le filtrat désacidifié et déminéralisé riche en oses, notamment en pentoses, peut être valorisé de différentes manières.

L'un des pentoses présent dans ledit filtrat, le xylose, peut être soumis à une transformation 5, celle-ci pouvant être biologique, chimique, ou enzymatique, de sorte à permettre la production de xylitol 18, ou encore d'intermédiaires chimiques, de tensio-actifs, ou de polymères 19.

Pour ces applications, il est nécessaire de séparer les sucres entre eux pour obtenir une solution de xylose pur. L'obtention de cette solution est réalisable par exemple en effectuant une chromatographie d'échange de ligand, une cristallisation ou une combinaison de ces deux techniques.

Dans un autre exemple de réalisation tout aussi avantageux, le xylose est purifié et extrait à partir du filtrat sous forme solide 20, par concentration 6 dudit filtrat, suivi d'une étape de cristallisation 7 pour obtenir du xylose cristallisé 20. Suite à cette étape de cristallisation 7, on obtient également une liqueur sucrée 21 qui peut, de manière optionnelle, être soumise à une étape de chromatographie d'échange de ligand 8 afin de récupérer le xylose 20 susceptible d'être contenu dans ladite liqueur 21 en les séparant des autres sucres 23.

Une telle étape permet avantageusement d'optimiser le rendement du procédé d'extraction du xylose à partir de l'hémicellulose contenue au départ dans la biomasse 9.

L'intérêt du procédé selon l'invention de purification des oses, notamment des pentoses, est illustré dans l'exemple ci-dessous en relation avec les figures 2 et 3 jointes à la présente demande.

### Exemple : Purification d'un hydrolysat de son de blé obtenu après hydrolyse de l'acide sulfurique dilué

Le tableau 1 ci-dessous présente les caractéristiques de l'hydrolysat de son de blé qui a été étudié après une étape d'hydrolyse dudit son de blé au moyen d'une solution d'acide sulfurique (H₂SO₄) diluée à une concentration massique égale à 15g/L.

**Tableau 1 : Composition de l'hydrolysat acide**

| MS % | pH | Conductivité (mS/cm) | MES (g/L) | DO à 420 nm | H₂SO₄ (g/L) | Glucose (g/L) | Xylose (g/L) | Arabinose (g/L) |
|---|---|---|---|---|---|---|---|---|
| 8,2 | 1,2 | 34,5 | 1,5 | 2,2 | 11,5 | 9 | 18 | 8 |

Dans ce tableau, MS représente la fraction massique de la somme des matières en solution ou en suspension dans l'hydrolysat, DO à 420 nm représente la densité optique, ou l'absorbance, mesurée à une longueur d'onde de 420 nm et MES représente les matières en suspension.

Avant de procéder à la purification de l'hydrolysat, celui-ci soumis à une étape de centrifugation pour éliminer les matières en suspension. Après centrifugation, le culot de résidu solide obtenu représente environ 10% du volume centrifugé.

Le surnageant obtenu est ultrafiltré à une température de 40°C avec une membrane organique Alfa Laval UFX10 en polysulfone sur polypropylène, résistante aux pH acides et présentant un seuil de coupure de 10 000 Da (ou 10 kDA) et avec une pression transmembranaire d'environ 6 bars.

Les caractéristiques du surnageant (hydrolysat initial), du rétentat, et du perméat, ou filtrat, ont été mesurées et sont reprises dans le tableau 2 ci-dessous.

**Tableau 2 : Composition après ultrafiltration de l'hydrolysat acide**

| | Volume (L) | Brix (°B) | pH | Conductivité (mS/cm) | MES à pH 5 (g/L) | DO à 420 nm |
|---|---|---|---|---|---|---|
| Hydrolysat initial | 8 | 8,2 | 1,2 | 34,5 | 3,6 | 2,2 |
| Rétentat final | 1,8 | 11,6 | 1,4 | 30,2 | - | 6,5 |
| Perméat final | 6,2 | 7,0 | 1,3 | 34,5 | 0,0 | 0,5 |

On observe une forte diminution, dans le perméat final (ou filtrat) de l'absorption à 420 nm (DO). Cette dernière représente la présence des macromolécules organiques colorantes. La diminution de la DO à 420 nm traduit en conséquence l'élimination de ces macromolécules dans le perméat.

En outre, il a été mesuré la quantité de matières en suspension (MES) à pH 5 avant et après l'étape d'ultrafiltration, ledit pH ayant été ajusté au moyen d'ajout d'une solution de soude sur un échantillon de solution. Les résultats repris dans le tableau 2 montrent l'élimination quantitative de ces molécules qui auraient été susceptibles de précipiter au cours de l'étape ultérieure d'électrodialyse. En effet, dans le perméat final, il n'y a plus aucune trace de matières en suspension (0,0 g/L).

Il a déjà été évoqué la possibilité de réaliser l'étape d'ultrafiltration au moyen d'un filtre présentant un seuil de coupure inférieur à 10 000 Da. Toutefois, les résultats montrent que, dans cet exemple ce seuil de coupure est suffisant pour éliminer efficacement les matières en suspension et les macromolécules organiques, pour préserver ainsi durablement la performance des membranes de l'électrodialyseur et prévenir la précipitation de composés organiques au sein des compartiments de ce dernier.

Dans l'étape suivante, un pilote d'électrodialyse conventionnelle à deux compartiments a été utilisé pour récupérer l'essentiel du catalyseur acide, l'acide sulfurique (H₂SO₄). Les caractéristiques du pilote sont rassemblées dans le tableau 3 ci-dessous.

**Tableau 3 : Caractéristiques du pilote d'électrodialyse EUR2-10**

| | |
|---|---|
| Membranes anioniques | AMX-Sb |
| Membranes cationiques | CMX-Sb |
| Surface | 200 cm² |
| Saumure | H₂S0₄ à 0,5 g/L |
| Electrolyte | NaCl |
| Tension | 12V |

Deux phases d'électrodialyse ont été conduites. Les résultats obtenus après ces deux phases sont repris dans le tableau 4 ci-après.

La saumure obtenue après la 1^{ère} phase d'électrodialyse a été recyclée lors de la 2^{ème} phase afin d'atteindre la concentration en acide nécessaire pour que celui-ci soit recyclé dans un nouveau procédé d'hydrolyse de la biomasse lignocellulosique ; cette concentration d'acide est fixée entre 10 et 20 g/L.

### 1^{ère} phase d'électrodialyse

Après l'étape d'ultrafiltration évoquée plus haut, un volume de 2L d'hydrolysat, ou filtrat, a été traité avec 2 L de saumure dite « fraîche » ayant une concentration en acide sulfurique de 0,5g/L. L'utilisation d'une telle saumure présentant une conductivité supérieure à 5 mS/cm facilite l'étape d'électrodialyse.

L'évolution du pH, de la conductivité et de l'intensité du courant en fonction du temps sont représentées respectivement sur les figures 2A, 2B et 2C ci-jointes.

Les résultats obtenus montrent que le produit a été en grande partie déminéralisé après une durée de l'ordre de 20 min d'électrodialyse ; en effet, à partir de 20 min, on observe sur la figure 2A que la conductivité résiduelle dans le produit est inférieure à 1mS/cm. Cela se traduit en outre par une chute de l'intensité du courant.

Les résultats du tableau 4 ci-dessous montrent que, après 20 minutes d'électrodialyse, plus de 90% de l'acide sulfurique a été transféré dans la saumure. En ce qui concerne les sucres, plus de 98% sont restés dans le produit.

Par conséquent, il n'est pas nécessaire de traiter le produit plus de 20 min.

La saumure atteint au final un pH égal à 1,3 et sa teneur en acide est d'environ 0,12 eq/L, soit une concentration en acide sulfurique H₂SO₄ de 5,9 g/L.

**Tableau 4 : Composition des produits en fin d'électrodialyse**

| | Volume (L) | Brix (°B) | pH | Conductivité (mS/cm) | H₂SO₄ (g/L) | Glucose (g/L) | Xylose (g/L) | Arabinose (g/L) |
|---|---|---|---|---|---|---|---|---|
| Produit 1^{ère} ED | 2 | 5,5 | 3,1 | 0,5 | 0,8 | 7,5 | 15,4 | 7,3 |
| Saumure 1^{ère} ED | 2 | 1,4 | 1,3 | 27,1 | 5,9 | 0,1 | 0,2 | 0,1 |
| Produit 2^{ème} ED | 2,2 | 5,6 | 2,8 | 0,9 | 1,5 | 7,4 | 15,4 | 7,3 |
| Saumure 2^{ème} ED | 1,2 | 3,9 | 1,0 | 60,0 | 14,2 | 0,0 | 0,4 | 0,2 |

### 2ème phase d'électrodialyse

Un volume égal à 1,2 L de saumure précédente est utilisé avec 2,2 L d'hydrolysat ultrafiltré. L'évolution du pH, de la conductivité et de l'intensité de courant en fonction du temps sont représentées respectivement sur les figures 3A, 3B et 3C.

A nouveau, le produit a été déminéralisé au bout de 20 min d'électrodialyse, la conductivité résiduelle étant inférieure à 1 mS/cm. Aussi, une durée de 20 min est suffisante pour transférer plus de 90% de l'acide sulfurique dans la saumure et conserver plus de 98% du sucre dans le produit.

Après la 2^{ème} phase d'électrodialyse, la saumure a atteint un pH proche de 1 et sa teneur en acide est d'environ 0,29 eq/L, soit une concentration en acide sulfurique H₂SO₄ de 14,2 g/L.

Par conséquent, la saumure présente une concentration en acide sulfurique suffisante pour être recyclée lors de l'étape d'hydrolyse de la biomasse lignocellulosique.

Pour finir, un traitement de finition par échange d'ions est effectué pour éliminer les dernières traces de sels et d'acides. Le produit issu de la 2^{ème} électrodialyse est introduit à 3,6 BV/h (« bed volume » par heure) dans deux colonnes montées en série qui contiennent respectivement une résine cationique forte (LEWATIT S2528) et une résine anionique faible (LEWATIT S4328).

Au total, jusqu'à 30 BV de produit ont pu être traités à température ambiante avant que l'une des deux résines ne soit saturée.

Si l'on établit une comparaison avec le procédé de référence, dans lequel on procède à une neutralisation partielle ou complète de l'hydrolysat, on peut ainsi traiter à chaque cycle au moins 10 fois plus de produits. Par conséquent, il est possible, avec le procédé selon l'invention, d'économiser l'eau et les réactifs chimiques correspondants.

Ce traitement a permis de diviser par 5 la conductivité du produit (celle-ci étant au final de 0,17 mS/cm) et donc sa teneur en sels.

Enfin, un traitement de finition par lit mélangé et charbon actif a permis d'obtenir une solution de pentoses déminéralisée, avec une conductivité inférieure à 10 µS/cm et parfaitement décolorée.

## Revendications

1. Procédé de purification d'oses issus de l'hémicellulose provenant de biomasses lignocellulosiques, lesdits oses étant contenus dans un hydrolysat acide obtenu par hydrolyse partielle de biomasses lignocellulosiques au moyen d'au moins un catalyseur acide, ledit hydrolysat acide comportant en outre une matrice cellulosique, des résidus solides et/ou des matières en suspension, ledit procédé étant **caractérisé en ce qu'**il comporte les étapes suivantes :
- on élimine, de l'hydrolysat acide, la matrice cellulosique et les résidus solides et/ou les matières en suspension pour obtenir un hydrolysat clarifié ;
- sans aucun ajout de réactif chimique basique pour augmenter le pH, on soumet ledit hydrolysat clarifié à au moins une étape d'ultrafiltration et/ou à au moins une étape de nanofiltration au moyen d'une membrane organique présentant un seuil de coupure compris entre 100 et 50 000 Da et étant résistante à un pH acide de l'ordre de 1, de sorte à obtenir, d'une part, un filtrat contenant la majorité des pentoses et le catalyseur acide non neutralisé et, d'autre part, un rétentât contenant les espèces susceptibles de précipiter sous l'effet d'une augmentation du pH ;
- on traite ledit filtrat par au moins une étape d'électrodialyse de sorte à récupérer le catalyseur acide, dans une solution enrichie en acide, et à obtenir un filtrat désacidifié.

2. Procédé de purification selon la revendication précédente **caractérisé en ce que**, préalablement à la filtration, on élimine, dans un premier temps, la matrice cellulosique puis, dans un second temps, les résidus solides et/ou les matières en suspension.

3. Procédé de purification selon la revendication précédente, **caractérisé en ce qu'**on élimine les résidus solides et/ou les matières en suspension par décantation et/ou centrifugation et/ou filtration sur presse et/ou filtration sur membrane.

4. Procédé de purification selon l'une quelconque des revendications précédentes, **caractérisé en ce que** le catalyseur acide utilisé consiste en de l'acide sulfurique dont la concentration, dans l'hydrolysat acide, est avantageusement comprise entre 5 et 50 g/L.

5. Procédé de purification selon l'une quelconque des revendications précédentes, **caractérisé en ce que** le seuil de coupure du filtre utilisé lors de l'étape de filtration est compris entre 10 000 et 15 000 Da.

6. Procédé de purification selon l'une quelconque des revendications précédentes, **caractérisé en ce que**, lors de l'étape d'électrodialyse, on introduit, dans les compartiments de l'appareil d'électrodialyse où se concentre l'acide de l'eau acidifiée de sorte à avoir une conductivité supérieure ou égale à 5 mS/cm.

7. Procédé de purification selon l'une quelconque des revendications précédentes, **caractérisé en ce qu'**il comporte, après l'étape d'électrodialyse, une étape dans laquelle on déminéralise ledit filtrat désacidifié par électrodialyse et/ou par chromatographie et/ou par échange d'ions.

8. Procédé de purification selon l'une quelconque des revendications précédentes, **caractérisé en ce que** lesdits oses sont des pentoses.

9. Procédé de purification selon la revendication précédente, **caractérisé en ce que** lesdits pentoses consistent en du xylose et/ou en de l'arabinose.

10. Procédé de purification selon la revendication précédente, **caractérisé en ce que**, suite à l'étape de déminéralisation du filtrat désacidifié, on purifie le xylose par concentration du filtrat désacidifié et déminéralisé puis par cristallisation.

11. Procédé de purification selon la revendication précédente, **caractérisé en ce que**, suite à l'étape de cristallisation, on obtient une liqueur sucrée, comprenant du xylose et au moins un autre ose, et on procède à une étape de séparation pour séparer le xylose du (des) autre(s) ose (s) .

12. Procédé de purification selon la revendication précédente, **caractérisée en ce qu'**on effectue la séparation du xylose du (des) autres(s) ose(s) par une cristallisation, ou par une chromatographie d'échange de ligand combinée ou non avec une cristallisation.

## Patentansprüche

1. Verfahren zum Reinigen von Osen aus Hemicellulose, die aus Lignocellulose-Biomassen stammt, wobei die Osen in einem Säurehydrolysat enthalten sind, das durch teilweise Hydrolysieren von Lignocellulose-Biomassen mittels mindestens eines sauren Katalysators erhalten wird, wobei das Säurehydrolysat ferner eine Cellulosematrix, feste Rückstände und/oder Schwebstoffe umfasst, wobei das besagte Verfahren **dadurch gekennzeichnet ist, dass** es folgende Schritte umfasst:
- die Cellulosematrix und die festen Rückstände und/oder die Schwebstoffe werden aus dem Säurehydrolysat entfernt, um ein geklärtes Hydrolysat zu erhalten;
- ohne Zugabe eines basischen chemischen Reagens zum Erhöhen des pH-Werts wird das besagte geklärte Hydrolysat mindestens einem Schritt der Ultrafiltration und/oder mindestens einem Schritt der Nanofiltration mittels einer organischen Membran unterzogen, die eine Schnittschwelle zwischen 100 und 50.000 Da aufweist und gegenüber einem sauren pH-Wert in der Größenordnung von 1 beständig ist, sodass einerseits ein Filtrat, das die Mehrheit der Pentosen und den nicht neutralisierten sauren Katalysator enthält, und andererseits ein Retentat, das die Spezies enthält, die unter dem Einfluss einer Erhöhung des pH-Werts präzipitieren können, erhalten werden;
- das besagte Filtrat wird mit mindestens einem Schritt der Elektrodialyse behandelt, sodass der saure Katalysator in einer mit Säure angereicherten Lösung rückgewonnen und ein entsäuertes Filtrat erhalten wird.

2. Verfahren zum Reinigen nach dem vorhergehenden Anspruch, **dadurch gekennzeichnet, dass** vor der Filtration in einer ersten Phase die Cellulosematrix, dann in einer zweiten Phase die festen Rückstände und/oder die Schwebstoffe entfernt werden.

3. Verfahren zum Reinigen nach dem vorhergehenden Anspruch, **dadurch gekennzeichnet, dass** die festen Rückstände und/oder die Schwebstoffe durch Dekantieren und/oder Zentrifugieren und/oder Filtrieren auf einer Presse und/oder Filtrieren auf einer Membran entfernt werden.

4. Verfahren zum Reinigen nach irgendeinem der vorhergehenden Ansprüche, **dadurch gekennzeichnet, dass** der verwendete saure Katalysator aus Schwefelsäure besteht, deren Konzentration im Säurehydrolysat vorteilhafterweise zwischen 5 und 50 g/l liegt.

5. Verfahren zum Reinigen nach irgendeinem der vorhergehenden Ansprüche, **dadurch gekennzeichnet, dass** die Schnittschwelle des im Schritt des Filtrierens verwendeten Filters zwischen 10.000 und 15.000 Da liegt.

6. Verfahren zum Reinigen nach irgendeinem der vorhergehenden Ansprüche, **dadurch gekennzeichnet, dass** während dem Schritt der Elektrodialyse in die Kammern des Elektrodialysegeräts, in denen sich die Säure konzentriert, angesäuertes Wasser eingeführt wird, sodass eine Leitfähigkeit höher oder gleich 5 mS/cm erhalten wird.

7. Verfahren zum Reinigen nach irgendeinem der vorhergehenden Ansprüche, **dadurch gekennzeichnet, dass** es nach dem Schritt der Elektrodialyse einen Schritt umfasst, in dem das entsäuerte Filtrat durch Elektrodialyse und/oder durch Chromatographie und/oder durch Ionenaustausch demineralisiert wird.

8. Verfahren zum Reinigen nach irgendeinem der vorhergehenden Ansprüche, **dadurch gekennzeichnet, dass** die besagten Osen Pentosen sind.

9. Verfahren zum Reinigen nach dem vorhergehenden Anspruch, **dadurch gekennzeichnet, dass** die besagten Pentosen aus Xylose und/oder Arabinose bestehen.

10. Verfahren zum Reinigen nach dem vorhergehenden Anspruch, **dadurch gekennzeichnet, dass** nach dem Schritt der Demineralisierung des entsäuerten Filtrats die Xylose durch Konzentration des entsäuerten und demineralisierten Filtrats, danach durch Kristallisation gereinigt wird.

11. Verfahren zum Reinigen nach dem vorhergehenden Anspruch, **dadurch gekennzeichnet, dass** nach dem Schritt der Kristallisation eine süße Lauge erhalten wird, die Xylose und mindestens eine weitere Ose umfasst, und ein Schritt des Trennens durchgeführt wird, um die Xylose von der bzw. den weiteren Osen zu trennen.

12. Verfahren zum Reinigen nach dem vorhergehenden Anspruch, **dadurch gekennzeichnet, dass** die Trennung der Xylose von der bzw. den weiteren Osen durch eine Kristallisation oder durch eine mit einer Kristallisation kombinierte oder nichtkombinierte Ligandenaustausch-Chromatographie durchgeführt wird.

## Claims

1. A method for purifying oses derived from hemicellulose originating from lignocellulosic biomasses, said oses being contained in an acid hydrolysate obtained by partial hydrolysis of lignocellulosic biomasses by means of at least one acid catalyst, said acid hydrolysate including in addition a cellulosic matrix, solid residues and/or suspended matters, said method being **characterized in that** it includes the following steps:
- the cellulosic matrix and the solid residues and/or the suspended matters are removed from the acid hydrolysate, in order to obtain a clarified hydrolysate;
- without any addition of a basic chemical reagent to increase the pH, said clarified hydrolysate is subjected to at least one ultrafiltration step and/or to at least one nanofiltration step by means of an organic membrane having a cut-off threshold between 100 and 50 000 Da and being resistant to an acidic pH of about 1, so as to obtain, on the one hand, a filtrate containing the majority of pentoses and the non-neutralized acid catalyst and, on the other hand, a retentate containing the species likely to precipitate under the action of an increase in pH;
- said filtrate is treated with at least one electro-dialysis step so as to recover the acid catalyst in an acid-enriched solution and to obtain a de-acidified filtrate.

2. Purification method according to the preceding claim, wherein, prior to the filtration, the cellulosic matrix is removed in a first step, then the solid residues and/or suspended matters are removed in a second step.

3. Purification method according to the preceding claim, wherein the solid residues and/or the suspended matters are removed by sedimentation and/or centrifugation and/or filtration on a press and/or filtration on a membrane.

4. Purification method according to any one of the preceding claims, wherein the acid catalyst being used consists of sulfuric acid the concentration of which in the acid hydrolysate is advantageously between 5 and 50 g/L.

5. Purification method according to any one of the preceding claims, wherein the cut-off threshold of the filter used during the filtration step is between 10 000 and 15 000 Da.

6. Purification method according to any one of the preceding claims, wherein, during the electro-dialysis step, acidified water is introduced into the compartments of the electro-dialysis apparatus where the acid concentrates, so as to have a conductivity higher than or equal to 5 mS/cm.

7. Purification method according to any one of the preceding claims, wherein it includes, after the electro-dialysis step, a step in which said de-acidified filtrate is demineralized by electro-dialysis and/or by chromatography and/or by ion exchange.

8. Purification method according to any one of the preceding claims, wherein said oses are pentoses.

9. Purification method according to the preceding claim, wherein said pentoses consist of xylose and/or arabinose.

10. Purification method according to the preceding claim, wherein, following the step of demineralization of the de-acidified filtrate, the xylose is purified by concentration of the de-acidified and demineralized filtrate and then by crystallization.

11. Purification method according to the preceding claim, wherein, following the crystallization step, a sweet liquor is obtained comprising xylose and at least another ose, and a separation step is carried out to separate the xylose from the other ose(s).

12. Purification method according to the preceding claim, wherein the separation of the xylose from the other ose(s) is carried out by a crystallization or by a ligand-exchange chromatography, whether or not combined with a crystallization.
